(19) Europäisches Patentamt
European Patent Office
Office européen des brevets

(11) Veröffentlichungsnummer : **0 179 377**
**B1**

(12) **EUROPÄISCHE PATENTSCHRIFT**

(45) Veröffentlichungstag der Patentschrift : **20.01.88**

(51) Int. Cl.$^4$ : **C 25 B 3/02**, C 07 D311/76

(21) Anmeldenummer : **85112975.9**

(22) Anmeldetag : **12.10.85**

# (54) Verfahren zur Herstellung von 1-Alkoxyisochromanen und neue 1-Alkoxy-alkylisochromane.

(30) Priorität : **20.10.84 DE 3438568**

(43) Veröffentlichungstag der Anmeldung : **30.04.86 Patentblatt 86/18**

(45) Bekanntmachung des Hinweises auf die Patenterteilung : **20.01.88 Patentblatt 88/03**

(84) Benannte Vertragsstaaten : **BE CH DE FR GB IT LI**

(56) Entgegenhaltungen :
**EP-A- 0 053 261**
**EP-A- 0 063 608**
**CHEMICAL ABSTRACTS, Band 76, Nr.3, 17.Januar 1972, Columbus, Ohio, US; J.THIBAULT "Synthesis and study of the reactivity of substituted isochromanes" Seite 361**

(73) Patentinhaber : **BASF Aktiengesellschaft**
**Carl-Bosch-Strasse 38**
**D-6700 Ludwigshafen (DE)**

(72) Erfinder : **Steiniger, Michael, Dr.**
**Villenstrasse 29**
**D-6730 Neustadt (DE)**
Erfinder : **Halbritter, Klaus, Dr.**
**Schwarzwaldstrasse 19**
**D-6800 Mannheim (DE)**
Erfinder : **Degner, Dieter, Dr.**
**Kurpfalzstrasse 8**
**D-6701 Dannstadt-Schauernheim (DE)**

**EP 0 179 377 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von 1-Alkoxyisochromanen durch elektrochemische Oxidation der entsprechenden Isochromane in Gegenwart von Alkoholen sowie neue 1-Alkoxy-alkylisochromane.

1-Alkoxyisochromane lassen sich nach den Angaben in Ann. Chim. 1971, 270, durch Bromierung von Isochroman in Tetrahydrofuran und Umsetzung des so erhaltenen 1-Bromisochromans mit Natriumalkoholat herstellen. Da bei diesem zweistufigen Verfahren toxische Einsatzstoffe verwendet werden, war nach einem Verfahren zu suchen, daß es gestattet, 1-Alkoxyisochromane auf günstigere Weise zu gewinnen.

Es wurde nun gefunden, daß man Isochromane der allgemeinen Formel

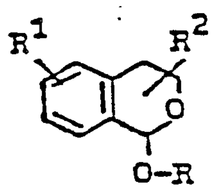

(I)

in der R für einen Alkylrest mit 1 bis 4 C-Atomen steht, $R^1$ und $R^2$ gleich oder verschieden sind und jeweils ein Wasserstoffatom oder eine unverzweigte oder verzweigte Alkylgruppe mit 1 bis 9 C-Atomen bedeuten, erheblich vorteilhafter dadurch herstellen kann, daß man ein Isochroman der allgemeinen Formel

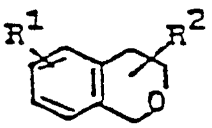

(II)

in der $R^1$ und $R^2$ die oben genannte Bedeutung haben, in Gegenwart eines Alkanols der Formel ROH, in der R die oben genannte Bedeutung hat, elektrochemisch oxidiert.

Die elektrochemische Oxidation läßt sich durch die folgenden Formeln wiedergeben :

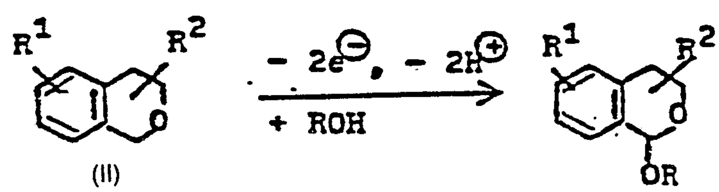


(II) (I)

Nach dem neuen Verfahren lassen sich die 1-Alkoxyisochromane der Formel I einstufig, mit hoher Regioselektivität und in guten Ausbeuten herstellen. Dabei ist es besonders überraschend, daß die Substitution ausschließlich an der benzylständigen Oxomethylengruppe (C-1 Position) eintritt. Mögliche Angriffe an weiteren Benzylpositionen, die man hätte erwarten können (s. J.C.S. Perkin I, 1978, 708) finden nicht statt.

In den Ausgangsstoffen der Formel II können die Reste $R^1$ und $R^2$ unverzweigte oder verzweigte Alkylgruppen mit 1 bis 9, vorzugsweise 1 bis 4 C-Atomen wie Methyl, Ethyl, Propyl, Isopropyl, Butyl, sek. Butyl oder tert. Butyl sein. Die Verwendung von Isochroman, 4-Methylisochroman und 5-Methylisochroman ist bevorzugt.

Die nach dem erfindungsgemäßen Verfahren erhältlichen 1-Alkoxy-alkylisochromane der Formel

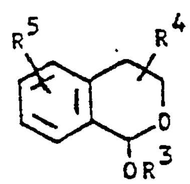

(III)

in der der Rest $R^3$ für Methyl oder Ethyl steht, einer der Reste $R^4$ oder $R^5$ eine Alkylgruppe mit 1 bis 4 C-Atomen und der andere ein Wasserstoffatom bedeutet oder beide Reste $R^4$ und $R^5$ Alkylgruppen mit 1 bis 4 C-Atomen bedeuten, sind neu.

Die Elektrooxidation wird in Gegenwart eines Alkanols der Formel ROH durchgeführt. R ist dabei eine

Alkylgruppe mit 1 bis 4 C-Atomen. Bevorzugt werden Methanol und Ethanol verwendet.

Das erfindungsgemäße Verfahren kann in den technischen üblichen Elektrolysezellen durchgeführt werden. Man verwendet vorzugsweise ungeteilte Durchflußzellen, die zweckmäßigerweise zur Minimierung der Zellspannung geringe Elektrodenabstände von beispielsweise 0,25 bis 2 mm ermöglichen. Als Anoden werden Edelmetalle, mit Edelmetalloxiden dotiertes Titan, Metalloxide, wie $MnO_2$ und $PbO_2$, bevorzugt jedoch Graphit eingesetzt. Die Kathodenmaterialien sind z. B. Stahl, Eisen, Nickel oder Graphit.

Die als Elektrolyt eingesetzte Lösung des Isochromans der Formel II in dem Alkanol ROH hat einen Gehalt an Isochroman von 1 bis 30, vorzugsweise 5 bis 20 Gew.-%. Zur Verbesserung der Leitfähigkeit enthält der Elektrolyt noch 0,1 bis 5, vorzugsweise 0,25 bis 2 Gew.-% eines Hilfselektrolyten. Als Hilfselektrolyte sind z. B. geeignet Basen, wie Alkalialkoholate, Alkalihydroxide, Neutralsalze wie Fluoride, Tetrafluoroborate, Sulfonate, und Sulfate und Säuren wie Alkylsulfonsäuren, Alkansulfonsäuren und Schwefelsäure. Besonders bevorzugt werden neutrale Hilfselektrolyte wie KF und $KSO_3C_6H_5$ und saure Hilfselektrolyte wie Schwefelsäure.

Die Elektrolysen werden mit 1 bis 6 F/Mol II durchgeführt, bevorzugt wird mit 2 bis 4 F/Mol II elektrolysiert. Die Stromdichten betragen 0,1 bis 20 $A/dm^2$, insbesondere 1 bis 8 $A/dm^2$. Man führt das erfindungsgemäße Verfahren z. B. bei Temperaturen durch, die zwischen 10 und 5 °C unter dem Siedepunkt des eingesetzten Alkanols liegen. Bevorzugt elektrolysiert man zwischen 20 und 40 °C. Die Elektrolyse kann sowohl diskontinuierlich als auch kontinuierlich durchgeführt werden.

Die Aufarbeitung der Elektrolyse erfolgt zweckmäßigerweise destillativ. Hierbei verfährt man z. B. so, daß man den überschüssigen Alkohol, gegebenenfalls nach Neutralisation mit einem Alkoholat, abdestilliert, die ausgefallenen Salze abfiltriert und das Produkt reindestilliert. Unumgesetzter Alkohol kann ohne weitere Reinigung zur Elektrolyse rückgeführt werden.

Die 1-Alkoxyisochromane der allgemeinen Formel I sind wichtige Zwischenprodukte, z. B. für die Herstellung von Isochromanyltropolonen, die Antitumoraktivitäten aufweisen (s. Chem. Pharm. Bull. 31, 2952 (1983)) oder von 4-Benzyl-1-(1-isochromanyl)piperidin, eine Verbindung, die die Freisetzung von Histamin inhibitiert und damit als Antiallergica wirkt. Die neuen 1-Alkoxyisochromane der Formel III zeigen fungizide Eigenschaften.

## Beispiel 1

Elektrosynthese von 1-Methoxyisochroman

Apparatur : ungeteilte Zelle mit 11 Graphitelektroden (Anode/Kathode : Graphit)

Elektrolyt :
291 g Isochroman
29 g $KSO_3C_6H_5$
2 590 g Methanol

Elektrolyse mit 3 F/Mol Isochroman
Stromdichte : 3,4 $A/dm^2$
Temperatur : 24 bis 26 °C
Durchfluß : 200 l/h

Aufarbeitung :

Nach Beendigung der Elektrolyse wird vom Elektrolyseaustrag Methanol bei Normaldruck und 65 bis 75 °C abdestilliert, das ausgefallene Salz bei Raumtemperatur über eine Drucknutsche abgetrennt (kann zusammen mit Methanol zur Elektrolyse rückgeführt werden) und das Filtrat bei 70 bis 80 °C und 0,3 mbar reindestilliert. Man erhält 16,8 g Isochroman und 252,9 g 1-Methoxyisochroman. Hieraus errechnen sich ein Umsatz von 98 %, eine Ausbeute von 71 % und eine Selektivität von 75 %.

## Beispiel 2

Elektrosynthese von 1-Ethoxyisochroman

Apparatur : ungeteilte Zelle mit 6 Graphitelektroden (Anode/Kathode : Graphit)

Elektrolyt :
559 g Isochroman
28 g Schwefelsäure
5 000 g Ethanol

Elektrolyse mit 2,5 F/Mol Isochroman
Stromdichte : 2 $A/dm^2$

Temperatur : 21 bis 23 °C
Durchfluß : 400 l/h

Aufarbeitung :

Nach Beendigung der Elektrolyse wird der Austrag neutralisiert und analog Beispiel 1 aufgearbeitet. Die Destillation erfolgt zwischen 70 und 100 °C und bei 0,3 mbar. Man erhält 16,4 g Isochroman und 488 g 1-Ethoxyisochroman. Hieraus errechnet sich ein Umsatz von 97 %, eine Ausbeute von 66 % und eine Selektivität von 68 %.

Beispiel 3

Elektrosynthese von 1-Methoxy-5-methylisochroman

Apparatur : ungeteilte Zelle mit 6 Graphitelektroden (Anode/Kathode : Graphit)

Elektrolyt :
888 g 5-Methylisochroman
29 g Schwefelsäure
5 000 g Methanol

Elektrolyse mit 2,6 F/Mol 5-Methylisochroman
Stromdichte : 3,4 $A/dm^2$
Temperatur : 22 bis 24 °C
Durchfluß : 400 l/h

Aufarbeitung :

Die Isolierung des Produktes erfolgt analog Beispiel 2. Man erhält 63 g 5-Methylisochroman und 580 g 1-Methoxy-5-methylisochroman. Hieraus errechnen sich ein Umsatz von 93 %, eine Ausbeute von 54 % und eine Selektivität von 58 %.

Beispiel 4

Elektrosynthese von 1-Methoxy-4-methylisochroman

Apparatur : ungeteilte Zelle mit 11 Graphitelektroden (Anode/Kathode : Graphit)

Elektrolyt :
270 g 4-Methylisochroman
27 g $KSO_3C_6H_5$
2 400 g Methanol

Elektrolyse mit 2,7 F/Mol 4-Methylisochroman
Stromdichte : 3,4 $A/dm^2$
Temperatur : 20 bis 22 °C
Durchfluß : 400 l/h

Aufarbeitung :

Der Austrag wird analog Beispiel 1 aufgearbeitet und das Rohprodukt bei 60 bis 80 °C und 0,1 mbar reindestilliert. Man isoliert 16 g 4-Methylisochroman und 223 g 1-Methoxy-5-methylisochroman als Diastereomerengemisch (1 : 1). Hieraus errechnen sich ein Umsatz von 94 %, eine Ausbeute von 69 % und eine Selektivität von 73 %.

**Patentansprüche**

1. Verfahren zur Herstellung von 1-Alkoxyisochromanen der allgemeinen Formel

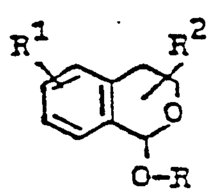

(I)

in der R für einen Alkylrest mit 1 bis 4 C-Atomen steht, und die Reste $R^1$ und $R^2$ gleich oder verschieden sind und jeweils ein Wasserstoffatom oder eine unverzweigte oder verzweigte Alkylgruppe mit 1 bis 9 C-Atomen bedeuten, dadurch gekennzeichnet, daß man ein Isochroman der allgemeinen Formel

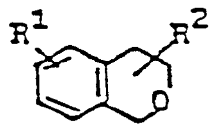

(II)

in der $R^1$ und $R^2$ die oben genannte Bedeutung haben, in Gegenwart eines Alkanols der Formel ROH, in der R die oben genannte Bedeutung hat, elektrochemisch oxidiert.

2. Verfahren nach Anspruch 1, dadurch gekennzeichnet, daß man für die Elektrooxidation als Elektrolyten eine Lösung verwendet, die 1 bis 30 Gew.-% des Isochromans der Formel II und 0,1 bis 5 Gew.-% eines Hilfselektrolyten in dem Alkanol enthält.

3. Verfahren gemäß Anspruch 2, dadurch gekennzeichnet, daß man neutrale oder saure Hilfselektrolyte verwendet.

4. Verfahren gemäß Ansprüchen 2 und 3, dadurch gekennzeichnet, daß man als Hilfselektrolyt eine Verbindung der Formeln KF, $KSO_3C_6H_5$, $H_2SO_4$, $CH_3SO_3H$ oder $C_6H_5SO_3H$ einsetzt.

5. 1-Alkoxyisochromane der Formel

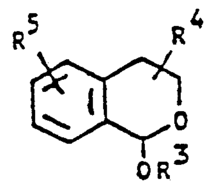

(III)

in der der Rest $R^3$ für Methyl oder Ethyl steht, einer der Reste $R^4$ oder $R^5$ eine Alkylgruppe mit 1 bis 4 C-Atomen und der andere ein Wasserstoffatom bedeutet oder beide Reste $R^4$ und $R^5$ Alkylgruppen mit 1 bis 4 C-Atomen bedeuten.

6. 1-Methoxy-5-methylisochroman.

7. 1-Methoxy-4-methylisochroman.

**Claims**

1. A process for the preparation of a 1-alkoxyisochroman of the general formula

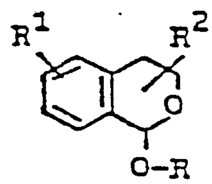

(I)

where R is alkyl of 1 to 4 carbon atoms, and the radicals $R^1$ and $R^2$ are identical or different and each is hydrogen, or linear or branched alkyl of 1 to 9 carbon atoms, wherein an isochroman of the general formula

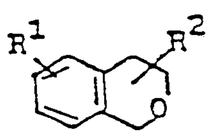

(II)

where $R^1$ and $R^2$ have the above meanings, is oxidized electrochemically in the presence of an alkanol of the formula ROH, where R has the above meaning.

2. A process as claimed in claim 1, wherein a solution containing from 1 to 30 % by weight of an isochroman of the formula II and from 0.1 to 5 % by weight of an auxiliary electrolyte in the alkanol is used as electrolyte for the electrooxidation.

3. A process as claimed in claim 2, wherein a neutral or acid auxiliary electrolyte is used.

4. A process as claimed in claims 2 and 3, wherein a compound of the formula KF, $KSO_3C_6H_5$, $H_2SO_4$, $CH_3SO_3H$ or $C_6H_5SO_3H$ is used as auxiliary electrolyte.

5. A 1-alkoxyisochroman of the formula

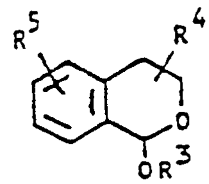

(III)

where $R^3$ is methyl or ethyl, one of the radical $R^4$ and $R^5$ is alkyl of 1 to 4 carbon atoms and the other radical is hydrogen, or the two radicals $R^4$ and $R^5$ are each alkyl of 1 to 4 carbon atoms.

6. 1-Methoxy-5-methylisochroman.

7. 1-Methoxy-4-methylisochroman.

**Revendications**

1. Procédé de préparation d'alcoxy-1 isochromanes de la formule générale

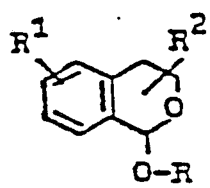

(I)

dans laquelle R désigne un radical alkyle en $C_1$ à $C_4$ et $R^1$ et $R^2$, qui peuvent être identiques ou différents, désignent chacun un atome d'hydrogène ou un radical alkyle en $C_1$ à $C_9$, ramifié ou non, caractérisé en ce que l'on soumet un isochromane de la formule générale

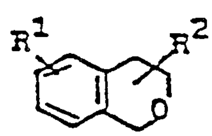

(II)

dans laquelle $R^1$ et $R^2$ possèdent les significations définies, à une oxydation électro-chimique en présence d'un alcanol de la formule ROH, dans laquelle R possède la signification définie.

2. Procédé suivant la revendication 1, caractérisé en ce que l'on emploie comme électrolyte pour l'électro-oxydation une solution contenant 1 à 30 % en poids d'un isochromane de la formule II et 0,1 à 5 % en poids d'un électrolyte auxiliaire dans l'alcanol.

3. Procédé suivant la revendication 2, caractérisé en ce que l'électrolyte auxiliaire est, soit neutre, soit acide.

4. Procédé suivant les revendications 2 et 3, caractérisé en ce que l'électrolyte auxiliaire est un composé choisi parmi ceux des formules KF, $KS_3C_6H_5$, $H_2SO_4$, $CH_3SO_3H$ et $C_6H_5SO_3H$.

5. Alcoxy-1 isochromanes de la formule

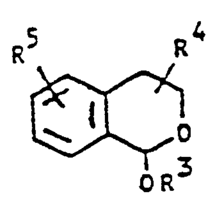

(III)

dans laquelle $R_3$ = méthyle ou éthyle et l'un des substituants $R^4$ et $R^5$ désigne un radical alkyle en $C_1$ à $C_4$ et l'autre un atome d'hydrogène ou les deux restes $R^4$ et $R^5$ représentent des radicaux alkyle en $C_1$ à $C_4$.

6. Le méthoxy-1 méthyl-5 isochromane.

7. Le méthoxy-1 méthyl-4 isochromane.